# EUROPEAN PATENT SPECIFICATION

(11) **EP 3 868 360 B1**
(45) Date of publication and mention of the grant of the patent: **10.12.2025**
(21) Application number: 19873258.8
(22) Date of filing: 15.10.2019
(51) Int. Cl.: A61K 8/49, A61Q 19/00, A61P 17/16, A61K 31/352

(54) **COMPOSITION FOR INHIBITING ACTIVATION OF MINERALCORTICOID RECEPTOR**
ZUSAMMENSETZUNG ZUR HEMMUNG DER AKTIVIERUNG EINES MINERALOCORTICOIDREZEPTORS
COMPOSITION POUR INHIBER L'ACTIVATION D'UN RÉCEPTEUR DES MINÉRALOCORTICOÏDES

(30) Priority: 15.10.2018 KR 20180122681
(43) Date of publication of application: 25.08.2021
(73) Proprietor: Amorepacific Corporation, Seoul 04386 (KR)
(72) Inventor: CHOI, Eun Jeong, Yongin-si Gyeonggi-do 17074 (KR); SON, Euidong, Yongin-si Gyeonggi-do 17074 (KR); KANG, Young Gyu, Yongin-si Gyeonggi-do 17074 (KR); KIM, Hyoung June, Yongin-si Gyeonggi-do 17074 (KR); LEE, Tae Ryong, Yongin-si Gyeonggi-do 17074 (KR)
(74) Representative: Schön, Christoph
(86) International application number: PCT/KR2019/013521
(87) International publication number: WO 2020/080801

(56) References cited:
- WO-A1-2007/000192
- WO-A2-02/089757
- WO-A2-2010/058936
- JP-A- 2001 213 775
- KR-A- 20100 055 734
- KR-A- 20100 055 734
- KR-A- 20150 043 029
- KR-A- 20160 081 192
- KR-A- 20160 095 721
- KR-B1- 101 893 323

## Description

### [Technical Field]

This disclosure relates to a non-therapeutic use of a composition for inhibiting activation of mineralocorticoid receptor.

### [Background Art]

Stress has been called a root of all illnesses since ancient times, and particularly in modern society, stress has excessively occurred because of various reasons such as social factors of study, work, marriage, parenting, and the like, and environmental factors such as weather, traffic, and the like for anyone regardless of sex or age, so it is recognized as a very serious social problem.

As our society has rapidly developed and diversified, roles required of modern people are increased, so that people suffering generalized anxiety disorders and mental disorders caused by many types of stress have increased. According to "A Study on Epidemiology of Mental Illness in 2006" published by the Ministry of Health and Welfare, "a one-year prevalence of metal illness" which refers to a percentage of people that experienced at least one type of mental illness for the year of 2006 was found to be 17.1%. This is around one person per 6 adults from greater than or equal to 18 years old to less than or equal to 64 years old, and 'a lifetime prevalence of metal illness' which is a percentage of people who experienced at least one type of mental illness for a whole life at the moment in 2006 was found to be 30%, which is one person per 3 adults. Considering the tendency toward increasing mental illness of adolescents caused by excessive academic enthusiasm or many types of stress, the prevalence for the entire population may be considered higher than the above.

Nowadays, anxiety disorder is treated by drug treatment along with a long-term psychotherapy in a clinic, and in the case of drug treatment, benzodiazepine-based anti-anxiety drugs such as diazepam, lorazepam, clonazepam, and alprazolam are mainly used, and azapirone-based buspirone is used as a drug for selectively acting on a serotonin receptor to selectively mitigate anxiety symptoms. In addition, recently, researches on stress controlling materials derived from natural materials capable of compensating side effects of these drugs have been actively performed.

The present inventors continuously researched materials derived from natural materials capable of preventing or treating mental stress-related diseases, and at last, confirmed that cortisol having an increased concentration in the epidermis under the mental stress condition is compatibly bonded to a mineralocorticoid receptor to deteriorate a skin barrier function. Further, it is also confirmed that skin barrier function deterioration is completely different in the mechanism from symptoms of other reasons which are not caused by mental stress (e.g., physical damage, aging, etc.). Furthermore, it is confirmed that a specific compound extracted from soy beans inhibits compatible binding of cortisol having an increased concentration in the epidermis to the mineralocorticoid receptor, so that one aspect of the present disclosure was completed.

Furthermore, a material capable of preventing or treating deterioration of a skin barrier function by suppressing activation of the mineralocorticoid receptor activated by binding cortisol having an increased concentration in the epidermis caused by mental stress has never been known.

International patent application WO-A-2010/058936 discloses skin external compositions used for inhibiting epidermal hyperproliferation and alleviating inflammatory skin diseases, comprises 4',6,7-trihydroxyisoflavone and/or 3',4',7-trihydroxyisoflavone. International patent application WO-A-02/089757 discloses the use of substances such as mono-hydroxy-isoflavone, mono-hydroxy-dihydro-isoflavone, dihydro-daidzein (dihydro-7,4'-dihydroxy-isoflavone), tetrahydro-daidzein (tetrahydro- 7,4'-dihydroxy-isoflavone), in cosmetic or dermatological preparations in the treatment and prophylaxis of symptoms of inflammatory or itchy skin conditions in sensible skin and in modifications of DNS synthesis and/or DNS repair in skin.

Korean Patent application KR-A-2010/005573 discloses a skin composition for external use containing 7,3,'4'-trihydroxyisoflavone to induce differentiation of keratinocytes and to enhance skin moisturization and to reinforce skin barrier; the composition contains 0.001-10 weight% of 7,3',4'-trihydroxyisoflavone as an active ingredient.

### [Disclosure]

### [Technical Problem]

The technical problem to be solved may be seen in providing a (cosmetic) composition capable of shortening a time for recovering a barrier function of a horny layer by inhibiting activation of mineralocorticoid receptor activated by binding cortisol which is a factor in deteriorating a skin barrier function caused by mental stress.

### [Technical Solution]

Subject matter of the present invention is a non-therapeutic use of a composition for inhibiting mineralocorticoid receptor activity including a compound represented by Chemical Formula 1 as an active ingredient as defined in the claims.

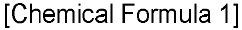

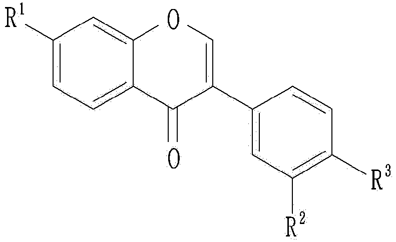

In Chemical Formula 1,
R¹ to R³ are each independently a hydrogen atom, a hydroxy group, or a substituted or unsubstituted C1 to C20 alkyl group.
R¹ to R³ may each independently be a hydroxy group.

### [Advantageous Effects]

According to an embodiment, a time for recovering a barrier function of a horny layer may be shortened by inhibiting activation of the mineralocorticoid receptor activated by binding cortisol which is a factor in deteriorating a skin barrier function caused by mental stress. In other words, it may specifically prevent and treat deterioration of a skin barrier function caused by mental stress which is one of various reasons of deteriorating a skin barrier function.

### [Description of the Drawings]

FIG. 1 is a photograph showing levels of gene expression of a glucocorticoid receptor and a mineralocorticoid receptor.
FIG. 2 is a graph showing a degree of the mineralocorticoid receptor activity through luciferase promoter assay.

### [Best Mode]

Hereinafter, embodiments of one aspect of the present disclosure will be described in detail, and may be easily performed by a person having ordinary skill in the related art.

In the present specification, the improvement of a skin barrier function is inhibiting compatibly binding of cortisol having an increased concentration in the epidermis caused by mental stress to a mineralocorticoid receptor thereby decreasing an activation level of the mineralocorticoid receptor, which is irrelevant in suppressing an oxidation stress or maintaining skin homeostasis and the like caused by physical damage or aging or the like. This is because the suppressing oxidation stress or the maintaining skin homeostasis and the like are not caused by mental stress, so the mechanism is totally different.

In addition, in the present specification, the mental stress is not a neurosis as referred to in a medical field, which is a maladapted status since a patient cannot adaptively adjust to psychological stress, but is a status of well adjusting to psychological stress but activating a mineralocorticoid receptor by compatibly binding of cortisol having an increased concentration in the epidermis to the mineralocorticoid receptor regardless of the will of the parts concerned.

In the present specification, when specific definition is not otherwise provided, "substituted" refers to replacement of at least one hydrogen by at least one substituent selected from a halogen atom (F, Cl, Br, or I), a hydroxy group, a C1 to C20 alkoxy group, a nitro group, a cyano group, an amine group, an imino group, an azido group, an amidino group, a hydrazino group, a hydrazono group, a carbonyl group, a carbamyl group, a thiol group, an ester group, an ether group, a carboxyl group or a salt thereof, a sulfonic acid group or a salt thereof, a phosphoric acid or a salt thereof, a C1 to C20 alkyl group, a C2 to C20 alkenyl group, a C2 to C20 alkynyl group, a C6 to C20 aryl group, a C3 to C20 cycloalkyl group, a C3 to C20 cycloalkenyl group, a C3 to C20 cycloalkynyl group, a C2 to C20 heterocycloalkyl group, a C2 to C20 heterocycloalkenyl group, a C2 to C20 heterocycloalkynyl group, a C3 to C20 heteroaryl group, or a combination thereof.

**In** the present specification, it will be understood that when an element such as a layer, film, region, or substrate is referred to as being "on" another element, it may be directly on the other element or intervening elements may also be present. In contrast, when an element is referred to as being "directly on" another element, there are no intervening elements present.

In the present specification, when a definition is not otherwise provided, the term "combination" refers to mixing or copolymerization. Also, "copolymerization" refers to block copolymerization or random copolymerization, and "copolymer" refers to a block copolymer or a random copolymer.

Hereinafter, a composition for inhibiting activation of mineralocorticoid receptor according to an embodiment is described.

The composition for inhibiting activation of mineralocorticoid receptor according to an embodiment includes a compound represented by Chemical Formula 1 as an active ingredient.

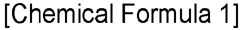

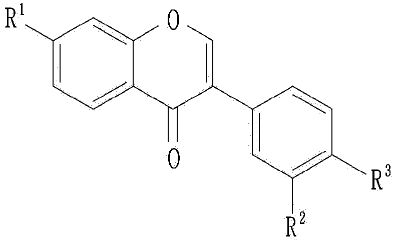

In Chemical Formula 1,
R¹ to R³ are each independently a hydrogen atom, a hydroxy group, or a substituted or unsubstituted C1 to C20 alkyl group.

For example, in Chemical Formula 1, R¹ to R³ may each independently be a hydroxy group.

The compound represented by Chemical Formula 1 inhibits binding of cortisol having an increased concentration in the epidermis under the mental stress condition to a mineralocorticoid receptor, providing effects of preventing activation of the mineralocorticoid receptor, so that the composition according to an embodiment may prevent deterioration of a skin barrier function even under the mental stress condition or rapidly improve the skin barrier function that is weakened by mental stress.

Specifically, under mental stress, wound healing is delayed, and the skin barrier function is deteriorated to reduce firmness of a horny layer or to delay recovery of a barrier function after damage, and specifically, under the mental stress condition, the concentration of cortisol (activated GC form) in a peripheral tissue is increased by increasing release of glucocorticoids (GC) through activation of the hypothalamus pituitary adrenal axis and increasing activation of a cortisol reductase (11β-hydroxysteroid dehydrogenase 1) in a peripheral tissue. The cortisol is bonded to a glucocorticoid receptor (GR) and a mineralocorticoid receptor (MR) which are receptors in the peripheral tissue in similar compatibility, causing many symptoms due to stress, and the representative symptom of the many symptoms is precisely deterioration of a skin barrier function. The composition according to an embodiment inhibits binding of cortisol having an increased concentration in the epidermis to a mineralocorticoid receptor and suppresses activation of the mineralocorticoid receptor, so that the deterioration of the skin barrier function may be prevented.

For example, the compound represented by Chemical Formula 1 may be a soybean extract. The compound represented by Chemical Formula 1 may be an extract of other materials besides soybeans, wherein the compound represented by Chemical Formula 1 derived from other materials besides soybeans is difficult to be employed for a cosmetic composition and moreover, does not play a role of inhibiting binding of cortisol having an increased concentration in the epidermis, related to mental stress, to the mineralocorticoid receptor. Further, when the compound represented by Chemical Formula 1 is an extract derived from soybeans, it may most effectively suppress activation of the mineralocorticoid receptor. Further, when the compound represented by Chemical Formula 1 is extracted from other materials besides soybeans, the mechanism is completely different compared to the case that the compound represented by Chemical Formula 1 is extracted from soybeans.

In other words, for enhancing a skin barrier under the mental stress condition, the compatible binding of cortisol having an increased concentration in the epidermis to the mineralocorticoid receptor is suppressed, so the compound represented by Chemical Formula 1 may inhibit binding of cortisol to the mineralocorticoid receptor, so as to prevent deterioration of the skin barrier function under the mental stress condition.

Thereby an embodiment provides a composition for inhibiting activation of a mineralocorticoid receptor including the compound represented by Chemical Formula 1 extracted from soybeans as an active ingredient, and may include a pharmaceutically effective amount of the compound represented by Chemical Formula 1 alone or may include at least one pharmaceutically acceptable carrier, excipient, or diluent.

The beans may include beans selected from soybeans, peas, and mung beans, germinated beans germinated from the beans, or a combination thereof. Specifically, the composition for inhibiting mineralocorticoid receptor activity according to an embodiment may include the compound represented by Chemical Formula 1 or natural product containing the compound represented by Chemical Formula 1 and its extract as an active ingredient, and the compound represented by Chemical Formula 1 or the natural products containing the compound represented by Chemical Formula 1 and its extract may be obtained from the beans selected from soybeans, peas, and mung beans, the germinated beans germinated from the beans, or the combination thereof. In addition, the extract of the natural product containing the compound represented by Chemical Formula 1 may be obtained by collecting an extract through cold precipitation at room temperature or warm precipitation of the natural product containing the compound represented by Chemical Formula 1 with 70% ethanol, completely concentrating it, dispersing it again in water, and fractionally collecting it again with at least one or two solvents selected from hexane, dichloromethane, chloroform, ethylacetate, butanol, ethanol, methanol, and water in the same amount. However, the extraction method may include all extraction methods of containing the compound represented by Chemical Formula 1 in a final extract.

The compound represented by Chemical Formula 1 may be included in a concentration range of 0.01 µg/ml to 1000 µg/ml, for example, a concentration range of 0.01 µg/ml to 100 µg/ml, in the composition. When the compound represented by Chemical Formula 1 is used as a cosmetic composition for inhibiting mineralocorticoid receptor activity, the compound represented by Chemical Formula 1 may be used at a concentration of greater than or equal to 0.01 µg/ml, greater than or equal to 0.1 µg/ml. The compound represented by Chemical Formula 1 may be used at a concentration of less than or equal to 1000 µg/ml, less than or equal to 100 µg/ml. When the compound represented by Chemical Formula 1 is used at a concentration of less than 0.01 µg/ml, the effect on inhibiting binding force of cortisol to the mineralocorticoid receptor is too negligible to provide the improvement of a skin barrier function; and when the compound represented by Chemical Formula 1 is used at a concentration of greater than 1000 µg/ml, it is not preferable since it is harmful to a human body because of cytotoxicity.

In the above, "pharmaceutically effective amount" refers to an amount sufficient to allow the physiologically active ingredient to be administered to an animal or human to exhibit desired physiological or pharmacological activity. However, the effective amount of the pharmaceutical may vary according to the degrees of symptoms, ages, weights, health status, sexes, administration routes, and duration of treatment.

In addition, "pharmaceutically acceptable" refers to physiologically acceptable when administered to humans, and usually does not cause allergic reactions or similar reactions, such as gastrointestinal disorders or dizziness. Examples of the carrier, excipient, and diluent may include lactose, dextrose, sucrose, sorbitol, mannitol, xylitol, erythritol, maltitol, starch, acacia rubber, alginate, gelatin, calcium phosphate, calcium silicate, cellulose, methyl cellulose, polyvinylpyrrolidone, water, methylhydroxybenzoate, propylhydroxybenzoate, talc, magnesium stearate, and mineral oils. In addition, it may further include fillers, anti-coagulants, lubricants, wetting agents, fragrances, emulsifiers, and antiseptics.

For example, the composition may be a cosmetic composition.

In the present specification "cosmetic" may refer to any material that may have a medical function in addition to the cosmetic function.

The formulation of the cosmetic composition may be appropriately selected as desired.

For example, the cosmetic composition may be formulated into formulations such as solutions, suspension liquids, emulsions, pastes, gels, creams, lotions, powders, soaps, surfactant-containing cleansings, oils, powder foundations, emulsion foundations, wax foundations, and sprays. More specifically, it may be formulated into cosmetic compositions such as detergents, tonics, hair dressings, nourishing lotions, essences, serums, treatments, conditioners, shampoos, lotions, wools, or hair dyes, and the like, and may be formulated into basic cosmetics such as an oil-in-water (O/W) type, a water-in-oil (W/O), and the like. In addition, in the composition, in addition to the above-mentioned essential components in each formulation, other components may be appropriately selected and formulated without difficulty by a person of ordinary skill in the art according to types or use purposes of other external preparations. For example, ultraviolet (UV) blocking agents, hair conditioning agents, fragrances, and the like may be further included.

The cosmetic composition may include a cosmetically acceptable medium or base. These are all formulations suitable for topical applications. The cosmetic composition may be provided in the form of emulsions obtained by dispersing an oil phase in an aqueous phase, suspensions, microemulsions, microcapsules, microgranules, or ion-type (liposome) and/or non-ionized vesicle dispersing agents, or in the form of creams, skins, lotions, powders, ointments, sprays, or conceal sticks. These compositions may be prepared according to conventional methods in the art.

When the formulation of one aspect of the present disclosure is a solution or emulsion, a solvent, a solubilizer, or an emulsifier may be used as carrier components. For example, water, ethanol, isopropanol, ethyl carbonate, ethyl acetate, benzyl alcohol, benzyl benzoate, propylene glycol, 1,3-butylglycol oil, glycerol aliphatic ester, polyethylene glycol, or fatty acid ester of sorbitan may be used.

If the formulation of one aspect of the present disclosure is a suspension, the carrier component may be a diluent of a liquid such as water, ethanol or propylene glycol, a suspending agent such as ethoxylated isostearyl alcohol, polyoxyethylene sorbitol ester, and polyoxyethylene sorbitan ester, microcrystalline cellulose, aluminum metahydroxide, bentonite, agar, tragacanth, and the like.

If the formulation of one aspect of the present disclosure is pastes, creams, or gels, the carrier component may be animal oil, vegetable oil, wax, paraffin, starch, tragacanth, cellulose derivatives, polyethylene glycol, silicone, bentonite, silica, talc or zinc oxide.

If the formulation of one aspect of the present disclosure is powders or sprays, the carrier component may be lactose, talc, silica, aluminum hydroxide, calcium silicate, or polyamide powders. Particularly, in the case of sprays, a propellant such as a chlorofluorohydrocarbon, propane/butane, or dimethyl ether may be additionally included.

In an embodiment of one aspect of the present disclosure, the cosmetic composition may include thickeners. The thickeners included in the cosmetic composition of one aspect of the present disclosure may be methyl cellulose, carboxyl methyl cellulose, carboxyl methyl hydroxy guanine, hydroxy methyl cellulose, hydroxyethyl cellulose, a carboxyl vinyl polymer, polyquaternium, cetearyl alcohol, stearic acid, and carrageenan, preferably one or more of carboxyl methyl cellulose, a carboxyl vinyl polymer, and polyquaternium may be used, and more preferably a carboxyl vinyl polymer may be used.

In an embodiment of one aspect of the present disclosure, the cosmetic composition may include a variety of suitable bases and additives as needed, and the types and amounts of these components may be easily selected by the inventor. If necessary, it may include an acceptable additive, and may further include for example, conventional ingredients such as antiseptics, pigments, additives, and the like.

The antiseptics may specifically be phenoxyethanol or 1,2-hexanediol, and the fragrances may be artificial fragrances.

In an embodiment of one aspect of the present disclosure, the cosmetic composition may include a composition selected from a water-soluble vitamin, an oil-soluble vitamin, a polymeric peptide, a polymeric polysaccharide, a sphingolipid, and a seaweed extract. Other ingredients that may be added include fats and oils, humectants, emollients, surfactants, organic and inorganic pigments, organic powders, ultraviolet (UV) absorbers, antiseptics, fungicides, antioxidants, plant extracts, pH adjusters, alcohols, pigments, fragrances, blood circulation accelerators, coolants, anhidrotics, purified water, and the like.

In addition, the compounding components which may be added other than these. Moreover, any component may be blended in the range which does not damage the purpose and effect of the invention.

Furthermore, the cosmetic composition according to an embodiment may be used not only as a pharmaceutical composition as described above, but also as a dietary supplement. For example, it may be easily used as main ingredients, auxiliary ingredients, food ingredients, food additives, functional foods, or beverages.

The "food" is a natural or processed product including one or more nutrients, and preferably is that it is ready to be eaten directly after a certain amount of processing. It includes all foods, food additives, functional foods, and beverages.

The foods to which the food composition can be added may include, for example, various foods, beverages, gums, teas, vitamin composites, and functional foods. In addition, the foods may include special nutritional products (e.g., formulas, baby food, etc.), processed meat products, fish products, tofu, jellies, noodles (e.g. ramen noodles, etc.), breads, dietary supplements, seasoned foods (e.g., soy sauce, soybean paste, red pepper paste, mixed soy sauce, etc.), sauces, sweets (e.g. snacks), candy, chocolate, gum, ice cream, dairy products (e.g. fermented milk, cheese, etc.), other processed foods, kimchi, pickles (various kimchi, pickles, etc.), beverages (e.g., fruit beverages, vegetable beverages, soy milk, fermented beverages, etc.), natural seasonings (e.g., ramen soup, etc.). The foods, beverages, or food additives may be prepared by conventional manufacturing methods.

In addition, "functional foods" or "health functional foods" refers to a food group that has added values to foods by using physical, biochemical, or biotechnological techniques to act and express functions of foods for specific purposes, or foods that are processed and designed to fully express the body's regulatory functions, such as defense rhythm control of food compositions, disease prevention, and recovery of living bodies. It may specifically be a health functional food. The functional food may include acceptable food auxiliary additives and may further include suitable carriers, excipients, and diluents commonly used in the manufacture of functional foods.

The types of dietary supplements may be in a form of powders, granules, tablets, capsules, or beverages.

According to another embodiment, provided is a method of inhibiting mineralocorticoid receptor activity by applying a composition including an effective amount of the compound represented by Chemical Formula 1 as an active ingredient, to the skin.

Advantages and features of one aspect of the present disclosure and methods for achieving them will be apparent with reference to the examples described in detail below. One aspect of the present disclosure will be described in detail with reference to examples. However, these examples are specifically provided for describing one aspect of the present disclosure.

### [Mode for Invention]

### (Examples)

### Experimental Example 1: Confirmation of Gene Expression of Mineralocorticoid Receptor

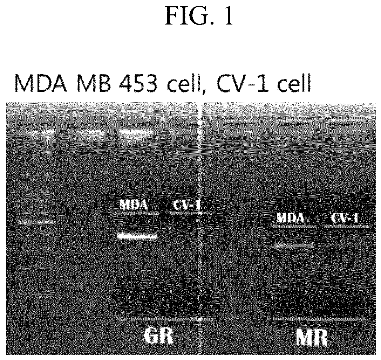

RNA was separated from a CV-1 cell and PCR was performed using reverse transcriptase, so gene expressions of a glucocorticoid receptor (GR) and a mineralocorticoid receptor (MR) were confirmed, and the results are shown in FIG. 1. As shown in FIG. 1, it is confirmed that only MR in the CV-1 cells was expressed, through this, it can be seen that the CV-1 cells are appropriate cells for confirming the effect of inhibiting activity of the mineralocorticoid receptor.

### Experimental Example 2: Inhibition Effect of Mineralocorticoid Receptor Activity

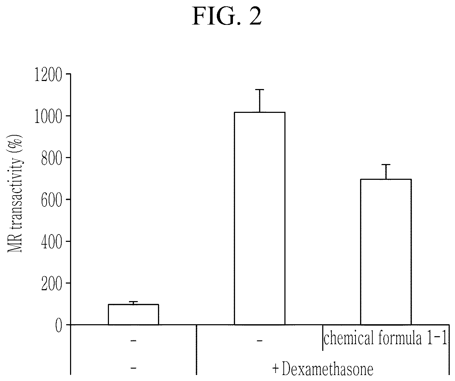

The CV-1 cell was cultivated in a 24-well flat incubator. After 24 hours, the incubating medium was replaced with a 10% charcoal dextran-treated FBS-DMEM, and then after 4 hours, a mixing DNA of a MMTV-luciferase reporter plasmid and an internal Control Group of a pRL-SV-40 plasmid was transfected using TransFast reagent (Promega). After 24 hours, it was treated with 10 µg /ml of the compound (INDOFINE Chemical Company, Inc.) represented by Chemical Formula 1-1, and after 2 hours, was treated with dexamethasone (1 nM), and then cultivated for 24 hours. The luciferase activity in the cell lysate was measured using a Dual-Luciferase Reporter Assay System (Promega), and the transfection efficiency was normalized by revising Renilla luciferase activity to measure a relative luciferase activity, and the results are shown in FIG. 2. As shown in FIG. 2, it is confirmed that the compound represented by Chemical Formula 1-1 deteriorated activity of the mineralocorticoid receptor by dexamethasone which is a kind of glucocorticoid.

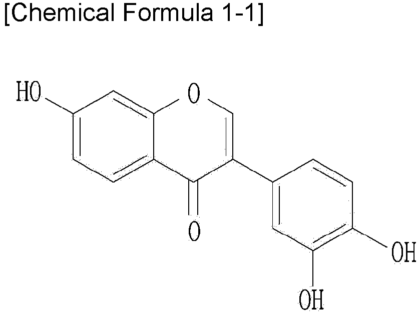

Although the preferred embodiments of one aspect of the present disclosure have been described in detail, and various modifications and improvements by those skilled in the art using the basic concept of one aspect of the present disclosure defined in the following claims are also within the scope of the invention.

## Claims

1. Non-therapeutic use of a composition for inhibiting activation of mineralocorticoid receptor, comprising
a compound represented by Chemical Formula 1 as an active ingredient: 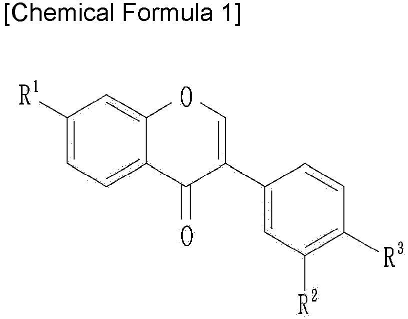
wherein, in Chemical Formula 1,
R¹ to R³ are each independently a hydrogen atom, a hydroxy group, or a C1 to C20 alkyl group.

2. The non-therapeutic use of claim 1, wherein R¹ to R³ are each independently a hydroxy group.

3. The non-therapeutic use of claims 1 or 2, wherein the compound represented by Chemical Formula 1 is obtained by extraction of soybeans.

4. The use of claims 1 to 3, wherein the composition further comprises at least one pharmaceutically acceptable carrier, excipient or diluent.

5. The non-therapeutic use of claims 1 to 4, wherein the compound represented by Chemical Formula 1 is included in the composition in a concentration range of 0.01 µg/ml to 1,000 µg/ml.

6. The non-therapeutic use of claims 1 to 5, wherein the composition is a cosmetic composition.

## Patentansprüche

1. Nicht-therapeutische Verwendung einer Zusammensetzung zur Hemmung der Aktivierung des Mineralocorticoidrezeptors, die als einen aktiven Inhaltsstoff eine Verbindung umfasst, die durch die chemische Formel 1 dargestellt ist: 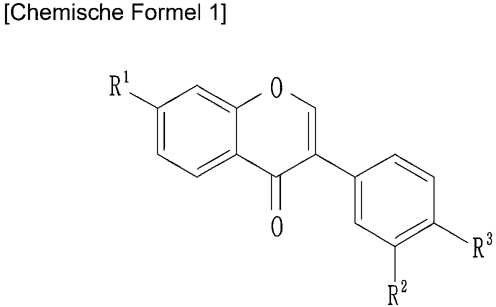 wobei in der chemischen Formel 1,
R¹ bis R³ jeweils unabhängig für ein Wasserstoffatom, eine Hydroxygruppe oder eine C1- bis C20-Alkylgruppe stehen.

2. Nicht-therapeutische Verwendung gemäß Anspruch 1, wobei R¹ bis R³ jeweils unabhängig für eine Hydroxygruppe stehen.

3. Nicht-therapeutische Verwendung gemäß den Ansprüchen 1 oder 2, wobei die durch die chemische Formel 1 dargestellte Verbindung durch Extraktion von Sojabohnen erhalten wird.

4. Verwendung gemäß den Ansprüchen 1 bis 3, wobei die Zusammensetzung des Weiteren mindestens einen pharmazeutisch akzeptablen Träger, Hilfsstoff oder Verdünnungsmittel umfasst.

5. Nicht-therapeutische Verwendung gemäß den Ansprüchen 1 bis 4, wobei die durch die chemische Formel 1 dargestellte Verbindung in der Zusammensetzung in einem Konzentrationsbereich von 0,01 µg/ml bis 1.000 µg/ml enthalten ist.

6. Nicht-therapeutische Verwendung gemäß den Ansprüchen 1 bis 5, wobei die Zusammensetzung eine kosmetische Zusammensetzung ist.

## Revendications

1. Usage non thérapeutique d'une composition pour inhiber l'activation d'un récepteur de minéralocorticoïde, comprenant
un composé représenté par la formule chimique 1 comme ingrédient actif : 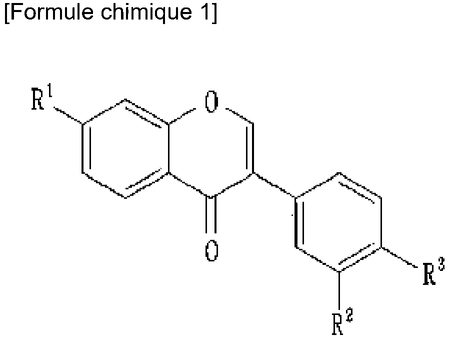
dans laquelle, dans la formule chimique 1,
R¹ à R³ sont chacun indépendamment un atome d'hydrogène, un groupe hydroxy ou un groupe alkyle en C1 à C20.

2. Usage non thérapeutique selon la revendication 1, dans lequel R¹ à R³ sont chacun indépendamment un groupe hydroxy.

3. Usage non thérapeutique selon les revendications 1 ou 2, dans lequel le composé représenté par la formule chimique 1 est obtenu par extraction de soja.

4. Usage selon les revendications 1 à 3, dans lequel la composition comprend en outre au moins un véhicule, excipient ou diluant pharmaceutiquement acceptable.

5. Usage non thérapeutique selon les revendications 1 à 4, dans lequel le composé représenté par la formule chimique 1 est compris dans une plage de concentration de 0,01 µg/ml à 1 0000 µg/ml.

6. Usage non thérapeutique selon les revendications 1 à 5, dans lequel la composition est une composition cosmétique.
